# EUROPEAN PATENT APPLICATION

(11) **EP 2 468 728 A1**
(43) Date of publication of application: **27.06.2012**
(21) Application number: 10382327.4
(22) Date of filing: 03.12.2010
(51) Int. Cl.: C07D 231/56, C07D 413/06, A61K 31/5377, A61K 31/416, A61P 29/00, A61P 25/00

(54) **2-(1-phenyl-4,5,6,7-tetrahydro-1H-indazol-4-YL)ethylamine derivatives useful as sigma receptor inhibitors**

(71) Applicant: Laboratorios del. Dr. Esteve, S.A., 08041 Barcelona (ES)
(72) Inventor: Corbera Arajona, Jordi, 08225 Terrassa ( Barcellona ) (ES)
(74) Representative: ABG Patentes, S.L.

(57) **Abstract**

The invention relates to compounds having pharmacological activity towards the sigma receptor, and more particularly to indazole derivatives of formula I 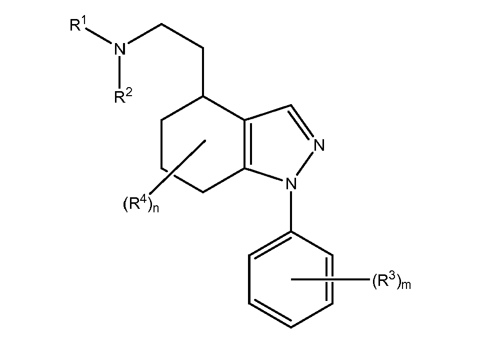 and to processes of preparation of such compounds, to pharmaceutical compositions comprising them, and to their use therapy and/or prophylaxis, in particular for the treatment of psychosis or pain.

## Description

### FIELD OF THE INVENTION

The present invention relates to compounds having pharmacological activity towards the sigma (σ) receptor, and more particularly to some indazole derivatives, to processes of preparation of such compounds, to pharmaceutical compositions comprising them, and to their use in therapy or prophylaxis, in particular for the treatment or prophylaxis of a sigma receptor mediated disease or condition.

### BACKGROUND

The search for new therapeutic agents has been greatly aided in recent years by better understanding of the structure of proteins and other biomolecules associated with target diseases. One important class of these proteins is the sigma (σ) receptor, a cell surface receptor of the central nervous system (CNS) which may be related to the dysphoric, hallucinogenic and cardiac stimulant effects of opioids. From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al, Pharmacological Reviews, 1990, 42, 355). It has been reported that the known sigma receptor ligand rimcazole clinically shows effects in the treatment of psychosis (Snyder, S.H., Largent, B.L. J. Neuropsychiatry 1989, 1, 7). The sigma binding sites have preferential affinity for the dextrorotatory isomers of certain opiate benzomorphans, such as (+)SKF 10047, (+)cyclazocine, and (+)pentazocine and also for some narcoleptics such as haloperidol.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al. Trends Pharmacol. Sci., 1992, 13:85-86). Most studies have implied that sigma binding sites (receptors) are plasmalemmal elements of the signal transduction cascade. Drugs reported to be selective sigma ligands have been evaluated as antipsychotics (Hanner, M. et al. Proc. Natl. Acad. Sci., 1996, 93:8072-8077). The existence of sigma receptors in the CNS, immune and endocrine systems have suggested a likelihood that it may serve as link between the three systems.

In view of the potential therapeutic applications of agonists or antagonists of the sigma receptor, a great effort has been directed to find selective ligands. Thus, the prior art discloses different sigma receptor ligands.

International patent application WO 2006/021463 discloses a family of selective inhibitors of the sigma receptor presenting a pyrazol group which is condensed with a cycloalkyl ring having 5, 6 or 7 carbon atoms. These compounds are encompassed within the following general formula:

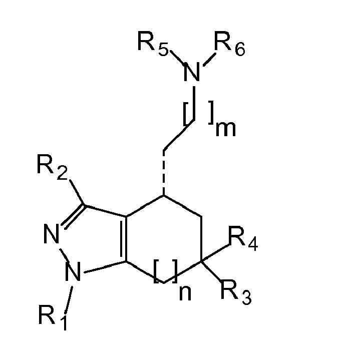

In spite of this background, there is still a need to find compounds that have pharmacological activity towards the sigma receptor, preferably being both effective and selective, and having good "drugability" properties, i.e. good pharmaceutical properties related to administration, distribution, metabolism and excretion.

### BRIEF DESCRIPTION OF THE INVENTION

The inventors of the present invention have surprisingly found a family of indazole derivatives which are particularly selective inhibitors of the sigma receptor. In particular, the compounds of the invention present an indazole framework substituted at position 4 by an ethyl chain containing an amine at its end and at position 1 by a phenyl ring.

Therefore, one aspect of the invention relates to compounds having the formula (I): 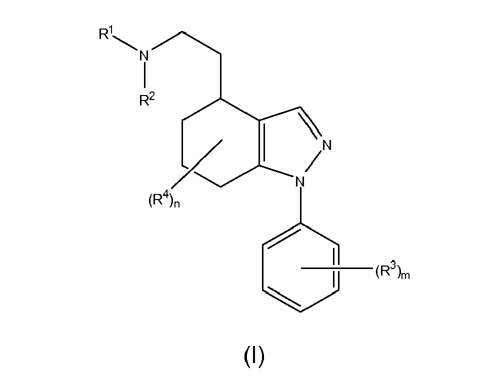 wherein
R¹ is selected from hydrogen or-CHO and R² is hydroxyethyl; or
R¹ and R² together with the nitrogen atom to which they are attached form a morpholinyl ring or a 2,3-dihydro-[1,4]oxazin-4-yl ring, said rings being optionally substituted with one or two groups selected from C₁₋₆alkyl, hydroxy, C₁₋₆alkoxy or oxo groups;
R³ is independently halogen, hydroxy or C₁₋₆alkoxy;
R⁴ is independently hydroxy or C₁₋₆alkoxy;
n and m are independently selected from 0, 1, and 2;
or a pharmaceutically acceptable N-oxide, salt, isomer, prodrug or solvate thereof;
with the proviso that the compound 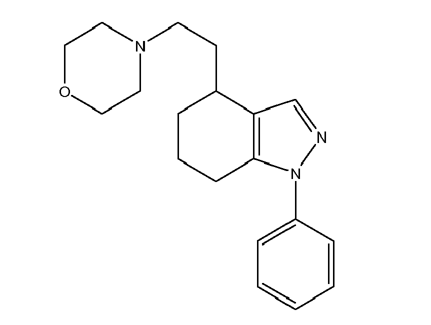 and its salts are excluded.

Another aspect of this invention refers to processes for the preparation of a compound of formula (I) as defined above or a pharmaceutically acceptable N-oxide, salt, isomer, prodrug or solvate thereof.

Another aspect of this invention refers to a medicament or pharmaceutical composition comprising at least one compound of formula (I) as defined above, or a pharmaceutically acceptable N-oxide, salt, isomer, prodrug or solvate thereof and a pharmaceutically acceptable carrier, adjuvant or vehicle.

Another aspect of this invention refers to a compound of formula (I) as defined above for use as a medicament, particularly for the treatment and/or prophylaxis of a sigma receptor-mediated disease or condition.

Another aspect of this invention refers to the use of a compound of formula (I) as defined above in the manufacture of a medicament for the treatment and/or prophylaxis of a sigma receptor-mediated disease or condition.

Another aspect of the present invention refers to a method for the treatment and/or prophylaxis of a sigma receptor-mediated disease or condition, the method comprising administering to the subject in need of such a treatment or prophylaxis a therapeutically effective amount of a compound of formula (I) as defined above.

In one embodiment, said sigma receptor-mediated disease or condition is selected from the group consisting of diarrhoea; migraine; obesity; elevated trigyceride levels; chylomicronemia; dysbetalipoproteinemia; hyperlipoproteinemia; hyperlipidemia; mixed hyperlipidemia; hypercholesterolemia; lipoprotein disorders; hypertriglyceridemia; sporadic hypertriglyceridemia; inherited hypertriglyceridemia and dysbetalipoproteinemia; arthritis; hypertension; arrhythmia; ulcer; learning, memory and attention deficits; cognition disorders; neurodegenerative diseases; demyelinating diseases; addiction to drugs and chemical substances including cocaine, amphetamine, ethanol and nicotine; tardive diskinesia; ischemic stroke; epilepsy; stroke; stress; cancer; psychotic conditions, in particular depression, anxiety or schizophrenia; inflammation; and autoimmune diseases.

In another embodiment, said sigma receptor-mediated disease or condition is pain, preferably neuropathic pain, inflammatory pain or other pain conditions involving allodynia and/or hyperalgesia.

In another embodiment, the compound of formula (I) is used as a pharmacological tool.

These aspects and preferred embodiments thereof are additionally also defined in the claims.

### DETAILED DESCRIPTION OF THE INVENTION

In the context of the present invention, the following terms have the meaning detailed below.

As used herein C₁₋₆alkyl, as a group or part of a group, defines straight or branched chain saturated hydrocarbon radicals having from 1 to 6 carbon atoms such as methyl, ethyl, n-propyl, i-propyl, n-butyl, t-butyl, pentyl, hexyl, and 2-methylbutyl. Likewise, C₁-₄alkyl, as a group or part of a group, defines straight or branched chain saturated hydrocarbon radicals having from 1 to 4 carbon atoms.

The term C₁₋₆alkoxy means C₁₋₆alkyloxy or a C₁₋₆alkyl ether radical, wherein the term C₁₋₆alkyl is as defined above. Likewise, the term C₁₋₄alkoxy means C₁₋₄alkyloxy or a C₁-₄alkyl ether radical, wherein the term C₁₋₄alkyl is as defined above. Examples of suitable alkyl ether radicals include methoxy, ethoxy, n-propoxy, iso-propoxy, n-butoxy, iso-butoxy, sec-butoxy, tert-butoxy, and hexanoxy.

The term oxo represents an oxygen atom doubly bonded i.e. =O.

It should be noted that the radical positions on any molecular moiety used in the definitions may be anywhere on such moiety as long as it is chemically stable.

Radicals used in the definitions of any variable herein include all possible isomers unless otherwise indicated. For instance, pentyl includes 1-pentyl, 2-pentyl and 3-pentyl.

The term "salt" must be understood as any form of an active compound used in accordance with this invention in which said compound is in ionic form or is charged and coupled to a counter-ion (a cation or anion) or is in solution. This definition also includes quaternary ammonium salts and complexes of the active molecule with other molecules and ions, particularly, complexes formed via ionic interactions. The definition includes in particular physiologically acceptable salts; this term must be understood as equivalent to "pharmacologically acceptable salts" or "pharmaceutically acceptable salts".

The term "pharmaceutically acceptable salts" in the context of this invention means any salt that is tolerated physiologically (normally meaning that it is not toxic, particularly, as a result of the counter-ion) when used in an appropriate manner for a treatment, applied or used, particularly, in humans and/or mammals. These physiologically acceptable salts may be formed with cations or bases and, in the context of this invention, are understood to be salts formed by at least one compound used in accordance with the invention -normally an acid (deprotonated)- such as an anion, particularly when used on humans and/or mammals. These physiologically acceptable salts may also be formed with anions or acids and, in the context of this invention, are understood as being salts formed by at least one compound used in accordance with the invention - normally protonated, for example in nitrogen - such as a cation and at least one physiologically tolerated anion, particularly when used on humans and/or mammals. This definition specifically includes in the context of this invention a salt formed by a physiologically tolerated acid, i.e. salts of a specific active compound with physiologically tolerated organic or inorganic acids - particularly when used on humans and/or mammals. Examples of this type of salts are those formed with: hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid.

The term "solvate" in accordance with this invention should be understood as meaning any form of the active compound in accordance with the invention in which said compound is bonded by a non-covalent bond to another molecule (normally a polar solvent), including especially hydrates and alcoholates, like for example, methanolate. A preferred solvate is the hydrate.

Any compound that is a prodrug of a compound of formula (I) is also within the scope of the invention. The term "prodrug" is used in its broadest sense and encompasses those derivatives that are converted in vivo to the compounds of the invention. Examples of prodrugs include, but are not limited to, derivatives and metabolites of the compounds of formula I that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger "Medicinal Chemistry and Drug Discovery 6th ed. (Donald J. Abraham ed., 2001, Wiley), "Design and Applications of Prodrugs" (H. Bundgaard ed., 1985, Harwood Academic Publishers) and Krogsgaard-Larsen et al. "Textbook of Drug design and Discovery" Taylor & Francis (April 2002).

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

Furthermore, any compound referred to herein may exist as tautomers. Specifically, the term tautomer refers to one of two or more structural isomers of a compound that exist in equilibrium and are readily converted from one isomeric form to another. Common tautomeric pairs are amine-imine, amide-imidic acid, keto-enol, lactam-lactim, etc.

Unless otherwise stated, the compounds of the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by ¹³C- or ¹⁴C-enriched carbon, or the replacement of at least one nitrogen by ¹⁵N-enriched nitrogen are within the scope of this invention.

The compounds of formula (I), or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

As noted previously, the term "pharmaceutically acceptable salts, solvates, prodrugs" refers to any salt, solvate, or any other compound which, upon administration to the recipient is capable of providing (directly or indirectly) a compound as described herein. However, it will be appreciated that non-pharmaceutically acceptable salts, solvates and prodrugs also fall within the scope of the invention since those may be useful in the preparation of pharmaceutically acceptable salts, solvates and prodrugs. The preparation of salts, solvates and prodrugs can be carried out by methods known in the art.

As used herein, the terms "treat", "treating" and "treatment" include the eradication, removal, reversion, alleviation, modification, or control of a sigma receptor-mediated disease or condition.

As used herein, the terms "prevention", "preventing", "preventive", "prevent" and "prophylaxis" refer to the capacity of a compound of formula (I) to avoid, minimize or difficult the onset or development of a sigma receptor-mediated disease or condition before its onset.

Therefore, by "treating" or "treatment" and "preventing" or "prevention", as a whole, is meant at least a suppression or an amelioration of the symptoms associated with the condition afflicting the subject, where suppression and amelioration are used in a broad sense to refer to at least a reduction in the magnitude of a parameter, e.g., symptom associated with the condition being treated. As such, the method of the present invention also includes situations where the condition is completely inhibited, e.g., prevented from happening, or stopped, e.g., terminated, such that the subject no longer experiences the condition.

As noted previously, the present invention relates to indazole compounds of general formula (I) having pharmacological activity towards the sigma (σ) receptor. It is stated that compound 4,5,6,7-tetrahydro-4-(2-(morpholin-4-yl)ethyl)-1-phenyl-1H-indazole (example 1 of International patent application WO 2006/021463) and its salts, in any enantiomeric purity (i.e. racemic mixtures, enantioenriched mixtures and enantiomers (R) and (S)), falling within the general formula (I), do not form part of the present invention.

According to one variant of the present invention, R¹ and R² together with the nitrogen atom to which they are attached form a morpholinyl ring or a 2,3-dihydro-[1,4]oxazin-4-yl ring, said rings being optionally substituted with one or two C₁₋₆alkyl, hydroxy, C₁-₆alkoxy or oxo groups. Thus, one embodiment of the invention relates to compounds having the formula (Ia): 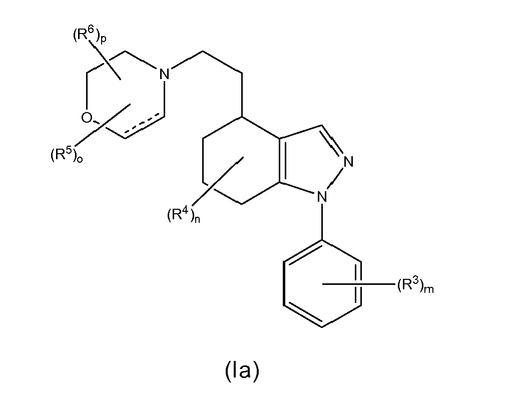 wherein
the dashed line (represented by - - - - -) represents an optional double bond;
R³ is independently halogen, hydroxy or C₁₋₆alkoxy;
R⁴ is independently hydroxy or C₁₋₆alkoxy;
R⁵ is independently C₁₋₆alkyl, hydroxy or C₁₋₆alkoxy;
R⁶ is oxo;
n, m and o are independently selected from 0, 1, and 2;
p is selected from 0, 1 and 2, with the proviso that o + p is ≤ 2; or a pharmaceutically acceptable N-oxide, salt, isomer, prodrug or solvate thereof; with the proviso that the compound 4,5,6,7-tetrahydro-4-(2-(morpholin-4-yl)ethyl)-1-phenyl-1 H-indazole and its salts are excluded.

The N-oxide compounds of the present compounds are meant to comprise the compounds of formula (I) wherein one or several nitrogen atoms are oxidized to the so-called N-oxide. As such, preferred compounds of formula (Ia) are selected from compounds of formula (Iaa): 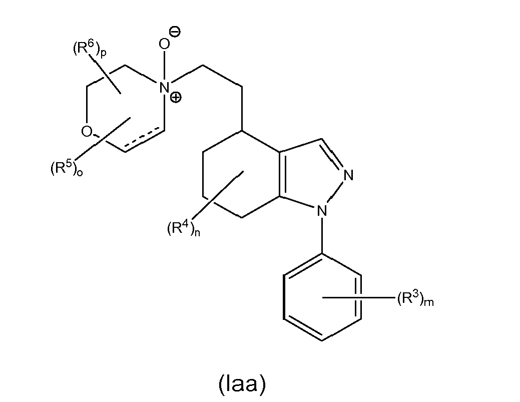 wherein
the dashed line (represented by - - - - -) represents an optional double bond;
R³ is independently halogen, hydroxy or C₁₋₆alkoxy;
R⁴ is independently hydroxy or C₁₋₆alkoxy;
R⁵ is independently C₁₋₆alkyl, hydroxy or C₁₋₆alkoxy;
R⁶ is oxo;
n, m and o are independently selected from 0, 1, and 2;
p is selected from 0, 1 and 2, with the proviso that o + p is ≤ 2;
or a pharmaceutically acceptable salt, isomer, prodrug or solvate thereof.

Compounds of formula (Iaa) wherein the dashed line (represented by - - - - -) is not present, i.e. there is no double bond resulting in a morpholinyl ring, are more preferred. One particular and preferred example is 4-(2-(1-phenyl-4,5,6,7-tetrahydro-1H-indazol-4-yl)ethyl)morpholine N-oxide

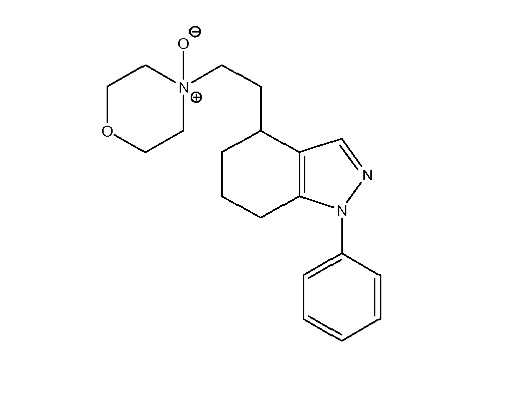

Other preferred compounds of formula (Ia) are selected from compounds of formula (lab): 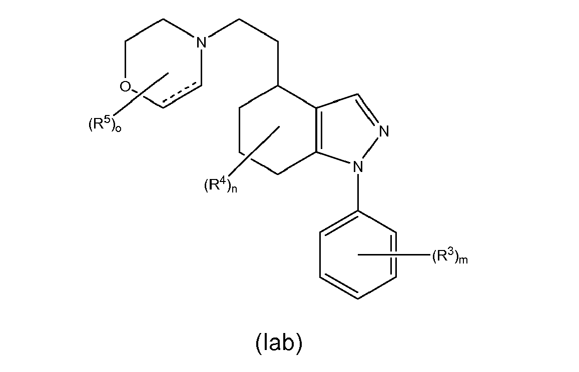 wherein
the dashed line (represented by - - - - -) represents an optional double bond;
R³ is independently halogen, hydroxy or C₁₋₆alkoxy;
R⁴ is independently hydroxy or C₁₋₆alkoxy;
R⁵ is independently C₁₋₆alkyl, hydroxy or C₁₋₆alkoxy;
n, m and o are independently selected from 0, 1, and 2, with the proviso that n + m + o is ≥ 1;
or a pharmaceutically acceptable salt, isomer, prodrug or solvate thereof.

Represetative subformulas falling within formula (lab) are depicted below:

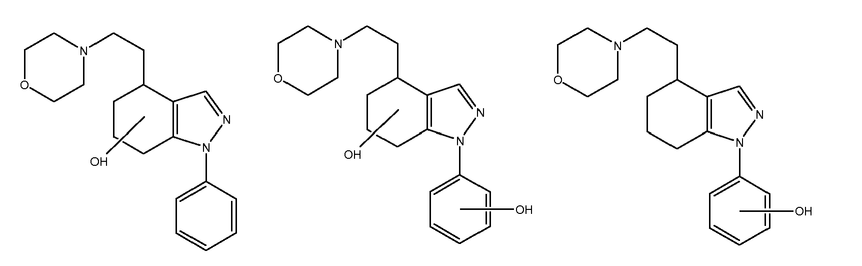

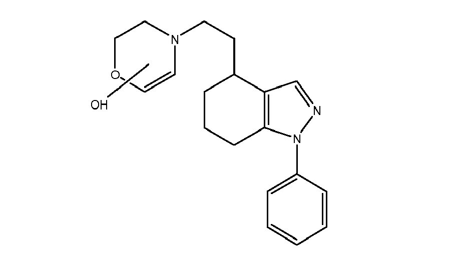

Further represetative subformulas falling within formula (lab) are depicted below:

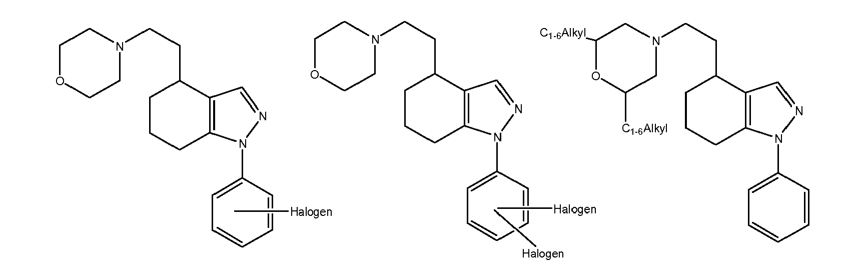

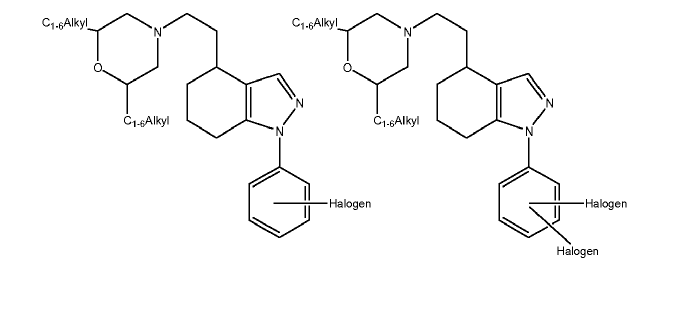

Preferred compounds of formula (Ia) are also selected from compounds of formula (lac): 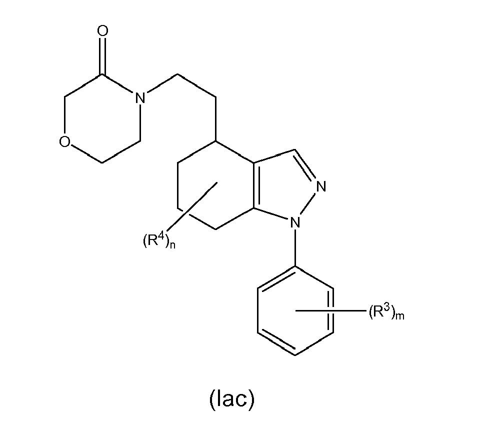 wherein
R³ is independently halogen, hydroxy or C₁₋₆alkoxy;
R⁴ is independently hydroxy or C₁₋₆alkoxy;
n and m are independently selected from 0, 1, and 2;
or a pharmaceutically acceptable salt, isomer, prodrug or solvate thereof.

According to another variant of the present invention, R¹ is selected from hydrogen or-CHO and R² is hydroxyethyl. Thus, a further embodiment of the invention relates to compounds having the formula (Ib): 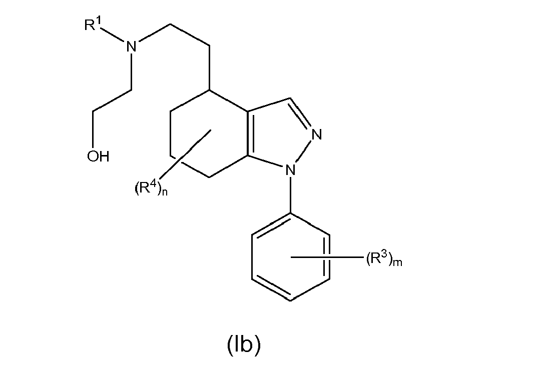 wherein
R¹ is selected from hydrogen or-CHO;
R³ is independently halogen, hydroxy or C₁₋₆alkoxy;
R⁴ is independently hydroxy or C₁₋₆alkoxy;
n and m are independently selected from 0, 1, and 2;
or a pharmaceutically acceptable N-oxide, salt, isomer, prodrug or solvate thereof.

Preferably, halogen is selected from fluoro or chloro in the above formulae. Likewise, C₁₋₄alkoxy is a preferred subgroup within C₁₋₆alkoxy; particular examples are methoxy, ethoxy and n-propoxy. Further, C₁₋₄alkyl is a preferred subgroup within C₁₋₆alkyl; particular examples are methyl, ethyl and n-propyl, preferably methyl.

In the compounds of formula (I) or subformulae thereof, the substituents R³, R⁴, R⁵ and -R⁶ may be bonded to any carbon atom of the corresponding ring.

In additional preferred embodiments, the preferences described above for the different substituents are combined. The present invention is also directed to such combinations of preferred substitutions in the formulae above.

Particular individual compounds of the invention falling under formula (I) include the compounds listed below:
- 4-(2-(1-phenyl-4,5,6,7-tetrahydro-1H-indazol-4-yl)ethyl)morpholine N-oxide;
- 4-(2-(1-(3,4-dichlorophenyl)-4,5,6,7-tetrahydro-1H-indazol-4-yl)ethyl)morpholine N-oxide;
- 2,6-dimethyl-4-(2-(1-phenyl-4,5,6,7-tetrahydro-1H-indazol-4-yl)ethyl)morpholine N-oxide;
- (2R,6S)-2,6-dimethyl-4-(2-(1-phenyl-4,5,6,7-tetrahydro-1H-indazol-4-yl)ethyl)morpholine N-oxide;
- 4-(2-(1-(4-fluorophenyl)-4,5,6,7-tetrahydro-1H-indazol-4-yl)ethyl)morpholine N-oxide;
- (R)-4-(2-(1-(4-fluorophenyl)-4,5,6,7-tetrahydro-1H-indazol-4-yl)ethyl)morpholine N-oxide;
- (S)-4-(2-(1-(4-fluorophenyl)-4,5,6,7-tetrahydro-1H-indazol-4-yl)ethyl)morpholine N-oxide;
- 4-(2-(1-(4-fluorophenyl)-4,5,6,7-tetrahydro-1H-indazol-4-yl)ethyl)-2,6-dimethylmorpholine N-oxide;
- (2R,6S)-4-(2-(1-(4-fluorophenyl)-4,5,6,7-tetrahydro-1H-indazol-4-yl)ethyl)-2,6-dimethylmorpholine N-oxide;
- 4-(2-Morpholin-4-yl-ethyl)-1-phenyl-4,5,6,7-tetrahydro-1H-indazol-5-ol;
- 4-(2-Morpholin-4-yl-ethyl)-1-phenyl-4,5,6,7-tetrahydro-1H-indazol-6-ol;
- 4-(2-Morpholin-4-yl-ethyl)-1-phenyl-4,5,6,7-tetrahydro-1H-indazol-7-ol;
- 1-(2-Hydroxy-phenyl)-4-(2-morpholin-4-yl-ethyl)-4,5,6,7-tetrahydro-1H-indazol-5-ol;
- 1-(3-Hydroxy-phenyl)-4-(2-morpholin-4-yl-ethyl)-4,5,6,7-tetrahydro-1H-indazol-5-ol;
- 1-(4-Hydroxy-phenyl)-4-(2-morpholin-4-yl-ethyl)-4,5,6,7-tetrahydro-1H-indazol-5-ol;
- 1-(2-Hydroxy-phenyl)-4-(2-morpholin-4-yl-ethyl)-4,5,6,7-tetrahydro-1H-indazol-6-ol;
- 1-(3-Hydroxy-phenyl)-4-(2-morpholin-4-yl-ethyl)-4,5,6,7-tetrahydro-1H-indazol-6-ol;
- 1-(4-Hydroxy-phenyl)-4-(2-morpholin-4-yl-ethyl)-4,5,6,7-tetrahydro-1H-indazol-6-ol;
- 1-(2-Hydroxy-phenyl)-4-(2-morpholin-4-yl-ethyl)-4,5,6,7-tetrahydro-1H-indazol-7-ol;
- 1-(3-Hydroxy-phenyl)-4-(2-morpholin-4-yl-ethyl)-4,5,6,7-tetrahydro-1H-indazol-7-ol;
- 1-(4-Hydroxy-phenyl)-4-(2-morpholin-4-yl-ethyl)-4,5,6,7-tetrahydro-1H-indazol-7-ol;
- 2-[4-(2-Morpholin-4-yl-ethyl)-4,5,6,7-tetrahydro-indazol-1-yl]-phenol;
- 3-[4-(2-Morpholin-4-yl-ethyl)-4,5,6,7-tetrahydro-indazol-1-yl]-phenol;
- 4-[4-(2-Morpholin-4-yl-ethyl)-4,5,6,7-tetrahydro-indazol-1-yl]-phenol;
- 4-[2-(1-Phenyl-4,5,6,7-tetrahydro-1H-indazol-4-yl)-ethyl]-3,4-dihydro-2H-[1,4]oxazin-2-ol;
- 4-[2-(1-Phenyl-4,5,6,7-tetrahydro-1H-indazol-4-yl)-ethyl]-3,4-dihydro-2H-[1,4]oxazin-3-ol;
- 4-[2-(1-Phenyl-4,5,6,7-tetrahydro-1H-indazol-4-yl)-ethyl]-5,6-dihydro-4H-[1,4]oxazin-2-ol;
- 4-[2-(1-Phenyl-4,5,6,7-tetrahydro-1H-indazol-4-yl)-ethyl]-5,6-dihydro-4H-[1,4]oxazin-3-ol;
- 4-[2-(1-Phenyl-4,5,6,7-tetrahydro-1H-indazol-4-yl)-ethyl]-morpholin-3-one;
- 2-[2-(1-Phenyl-4,5,6,7-tetrahydro-1H-indazol-4-yl)-ethylamino]-ethanol; and
- N-(2-Hydroxy-ethyl)-N-[2-(1-phenyl-4,5,6,7-tetrahydro-1H-indazol-4-yl)-ethyl]-formamide;
or a pharmaceutically acceptable N-oxide, salt, isomer, prodrug or solvate thereof.

Preferred individual compounds of the invention are (R) and (S)-4-(2-(1-phenyl-4,5,6,7-tetrahydro-1H-indazol-4-yl)ethyl)morpholine N-oxide as well as its salts, the hydrochloride salt being particularly preferred. Moreover, (S)-4-(2-(1-phenyl-4,5,6,7-tetrahydro-1H-indazol-4-yl)ethyl)morpholine N-oxide hydrochloride represents a more preferred embodiment.

The compounds of formula (I) defined above can be obtained by available synthetic procedures. For example, they can be prepared by the coupling of a compound of formula (II): 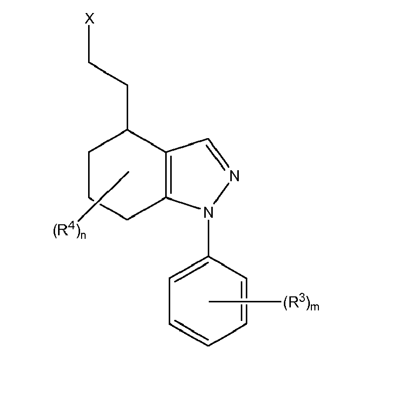 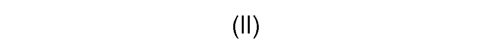 in which R³-R⁴, m and n are as defined above in formula (I), and X is a leaving group, preferably chlorine or pyridinium, with a compound of formula (III):

HNR¹R² (III)

in which R¹ and R² are as defined above in formula (I).

The reaction of compounds of formulas (II) and (III) is preferably carried out in an aprotic solvent, but not limited to, such as dimethylformamide (DMF) in the presence of an inorganic base, such as K₂CO₃. Compounds of formula (III) are commercially available or can be prepared by conventional methods.

The obtained compounds, when necessary, can be collected from the reaction mixture according to the methods known in the art. For example, when insoluble materials are present, the desired compound can be obtained -after removing the insoluble materials by filtration- by removing the solvent, e.g. by removing the solvent under reduced pressure, and/or by adding water to the residue and extracting the mixture with a water-immiscible organic solvent such as ethyl acetate, etc. Optionally, the desired compound can be obtained after drying over anhydrous sodium sulfate, for instance, and further, if necessary, by purifying with any conventional method, such as recrystallization, column chromatography, or other techniques.

It is evident that in the foregoing and in the exemplified reactions, the reaction products may be isolated from the reaction medium and, if necessary, further purified by methods generally known in the art, such as extraction, crystallization, trituration and chromatography. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution. Many compounds comprised by formula (I) may be converted into each other following functional group transformation reactions well known in the art. Preferably, they are obtained by utilizing suitable starting materials, like for example, compounds of formula (II) and (III) including already the desired substituents.

In particular, in those compounds with hydroxy substitutents as R³, R⁴ or R⁵, such hydroxy moieties may be converted into the corresponding C₁₋₆ alkoxy by reacting the compounds with an C₁₋₆alkyl halide in the presence of a base, such as an alkali of alkaline metal hydride, like lithium hydride or sodium hydride, or an alkali metal alkoxide, like sodium or potassium methoxide or ethoxide, potassium tert-butoxide, or potassium carbonate, triethylamine, pyridine, sodium iodide, cesium carbonate, etc. The C₁₋₆alkyl halide may be selected, for instance, from methyl or ethyl iodide.

In addition, in those compounds with C₁₋₆alkoxy substitutents as R³, R⁴ or R⁵, such C₁₋₆alkoxy moieties may be converted into the corresponding hydroxy by submitting the relevant compounds to acidic conditions, such as with hydrochloric acid, hydrobromic acid, or hydroiodic acid.

The compounds of formula (I) of the present invention may be converted to the corresponding N-oxide forms following procedures known in the art for converting a trivalent nitrogen into its N-oxide form. The N-oxide forms of the present compounds are meant to comprise the compounds of formula (I) wherein one or several nitrogen atoms are oxidized to the so-called N-oxide. Said N-oxidation reaction may generally be carried out by reacting compound of formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarbo-peroxoic acid or halo substituted benzenecarboperoxoic acid, e.g. 3-chlorobenzene-carboperoxoic acid, peroxoalkanoic acids, e.g. peroxoacetic acid, alkylhydroperoxides, e.g. tert-butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

It has been found that the compounds of general formula (I) have high affinity to sigma receptors, i.e. they are selective ligands for the sigma receptor and act as modulators, e.g. antagonists, inverse agonists or agonists, on these receptors.

The present invention further provides medicaments or pharmaceutical compositions comprising a compound of this invention, or a pharmaceutically acceptable N-oxide, salt, derivative, prodrug or stereoisomer thereof together with a pharmaceutically acceptable carrier, adjuvant, or vehicle, for administration to a patient.

The auxiliary materials or additives of a pharmaceutical composition according to the present invention can be selected among carriers, excipients, support materials, lubricants, fillers, solvents, diluents, colorants, flavour conditioners such as sugars, antioxidants, binders, adhesives, disintegrants, anti-adherents, glidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition.

The medicament or pharmaceutical composition according to the present invention may be in any form suitable for the application to humans and/or animals, preferably humans including infants, children and adults and can be produced by standard procedures known to those skilled in the art. Therefore, the formulation in accordance with the invention may be adapted for topical or systemic application, particularly for dermal, transdermal, subcutaneous, intramuscular, intra-articular, intraperitoneal, intravenous, intra-arterial, intravesical, intraosseous, intracavernosal, pulmonary, buccal, sublingual, ocular, intravitreal, intranasal, percutaneous, rectal, vaginal, oral, epidural, intrathecal, intraventricular, intracerebral, intracerebroventricular, intracisternal, intraspinal, perispinal, intracranial, delivery via needles or catheters with or without pump devices, or other application routes.

In a preferred embodiment the pharmaceutical compositions are in oral form, either solid or liquid. Suitable dose forms for oral administration may be tablets, pills, caplets, gel caps, chewing gums, capsules, granules, drops, syrups or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or reconstitutable dry preparations, aerosols or sprays in the apropriate unit dosage form. Adequate excipients can be used, such as bulking agents, buffering agents or surfactants.

The composition of the invention may be formulated as deposits in dissolved form or in patches, for percutaneous application.

Skin applications include ointments, gels, creams, lotions, suspensions or emulsions.

Suitable form of rectal application is by means of suppositories.

The mentioned formulations will be prepared using standard methods such as those described or referred to in the Spanish and US Pharmacopoeias and similar reference texts.

In one embodiment of the invention it is preferred that compound of formula (I) is used in therapeutically effective amounts. The physician will determine the dosage of the present therapeutic agents which will be most suitable and it will vary with the form of administration and the particular compound chosen, and furthermore, it will vary with the patient under treatment, the age of the patient, the type of disease or condition being treated. When the composition is administered orally, larger quantities of the active agent will be required to produce the same effect as a smaller quantity given parenterally. The compounds are useful in the same manner as comparable therapeutic agents and the dosage level is of the same order of magnitude as is generally employed with these other therapeutic agents. Active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

The following examples are merely illustrative of certain embodiments of the invention and cannot be considered as restricting it in any way.

### Examples

### Example 1: Synthesis of 4-(2-(1-phenyl-4,5,6,7-tetrahydro-1H-indazol-4-yl)ethyl)morpholine N-oxide.

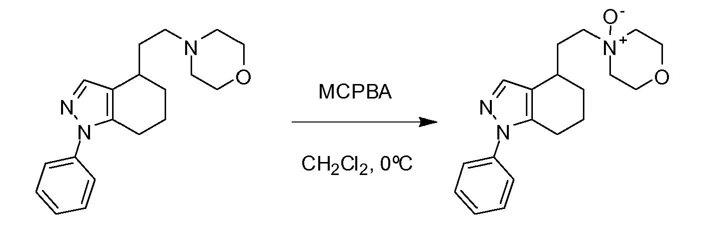

A solution of m-chloroperbenzoic acid (∼70% purity, 0.67 g, 2.75 mmol) in CH₂Cl₂ (40 mL) was dried over sodium sulphate and filtered and then was added to a solution of 4-(2-(1-phenyl-4,5,6,7-tetrahydro-1H-indazol-4-yl)ethyl)morpholine (0.78 g, 2.5 mmol) in CH₂Cl₂ (10 mL) cooled in an ice-bath. The mixture was allowed to reach room temperature and the reaction was monitored by TLC. The mixture was washed with a solution of NaOH and the aqueous phase was extracted with CH₂Cl₂ several times. The organic phase was dried with sodium sulphate, filtered and removed under reduced pressure. The resulting crude (0.32 g) was purified by flash chromatography (CH₂Cl₂: M e O H 8:2) to yield 4-(2-(1-phenyl-4,5,6,7-tetrahydro-1H-indazol-4-yl)ethyl)morpholine N-oxide (0.19 g, 0.60 mmol, 24%) as beige solid.
¹H NMR (300 MHz, CDCl₃) δ: 7.55 (s, 1 H), 7.48 (m, 4H), 7.34 (m, 1 H), 4.50 (t, 2H), 3.79 (d, 2H), 3.26-3.42 (m, 4H), 3.13 (t, 2H), 2.89 (m, 1 H), 2.72 (m, 2H), 2.43 (m, 1 H), 2.18 (m, 1 H), 1.99 (m, 2H), 1.72 (m, 1 H), 1.52 (m, 1 H).
¹H NMR (300 MHz, CDCl₃+ TFA) δ: 8.28 (s, 1 H), 7.63 (m, 3H), 7.42 ( d, J= 7.3 Hz, 2H), 4.28 (m, 2H), 4.10 (m, 2H), 3.80-4.00 (m, 4H), 3.70 (m, 2H), 2.98 (m, 1 H), 2.70 (m, 2H), 2.50 (m, 1 H), 2.02-2.32 (m, 3H), 1.80 (m, 1 H), 1.50 (m, 1 H).

### Example 2: 4-(2-(1-phenyl-4,5,6,7-tetrahydro-1H-indazol-4-yl)ethyl)morpholineN-oxide hydrochloride.

To a 4-(2-(1-phenyl-4,5,6,7-tetrahydro-1H-indazol-4-yl)ethyl)morpholine N-oxide (1.19 g, 3.64 mmol) dissolved in acetone (8 mL) was added HCl 4N in dioxane (0.91 mL) to yield 4-(2-(1-phenyl-4,5,6,7-tetrahydro-1H-indazol-4-yl)ethyl)morpholine N-oxide hydrochloride as a solid (0.40 g, 1.10 mmol, 30%).
Beige solid. m.p. 174.5-175.5°C
¹H NMR (300 MHz, DMSO-*d₆*) δ: 12.44 (br b, 1 H), 7.67 (s, 1 H), 7.52 (m, 4H), 7.35 (m, 1 H), 3.96 (m, 4H), 3.89 (m, 2H), 3.74 (m, 4H), 2.74 (m, 3H), 2.27 (m, 1 H), 1.93 (m, 3H), 1.64 (m, 1 H), 1.41 (m, 1 H).

### Example 3: Synthesis of (R)-4-(2-(1-phenyl-4,5,6,7-tetrahydro-1H-indazol-4-yl) ethyl)morpholine N-oxide.

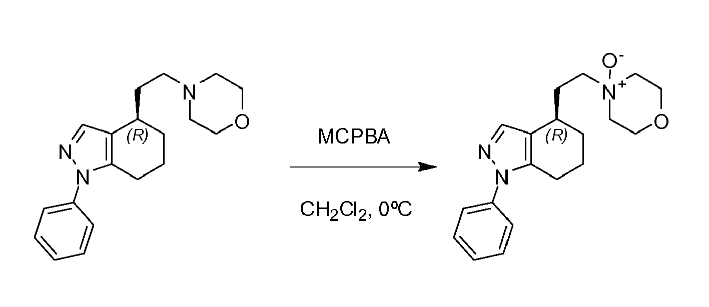

To a solution of (R)-4-(2-(1-phenyl-4,5,6,7-tetrahydro-1H-indazol-4-yl)ethyl)morpholine (390 mg, 1.25 mmol) in CH₂Cl₂ (20 mL) was added portionwise m-chloroperbenzoic acid (∼70% purity, 367 mg, 1.49 mmol) and stirred at room temperature for 4 hours, monitoring the reaction by TLC. The solution was washed twice with NaOH 1N and water and the organic phase was dried and removed under reduced pressure. The resulting crude was purified by flash chromatography to yield (R)-4-(2-(1-phenyl-4,5,6,7-tetrahydro-1H-indazol-4-yl)ethyl)morpholine N-oxide as an oil (171 mg, 0.52 mmol, 41.7%).
¹H NMR (300 MHz, CDCl₃) δ: 7.54 (s, 1 H), 7.48 (m, 4H), 7.33 (m, 1 H), 4.49 (t, *J* = 11.2 Hz, 2H), 3.79 (d, *J*= 12.3 Hz, 2H), 3.41 (m, 2H), 3.32 (td, *J*= 11.6, 3.5 Hz, 2H), 3.17 (t, *J* = 9.8 Hz, 2H), 2.90 (m, 1 H), 2.72 (m, 2H), 2.42 (m, 1 H), 1.73-2.09 (m, 3H), 1.52 (m, 1 H).
[α]²⁰₄₃₆ = -7.8 (c=1, MeOH)
Retention time = 16.78 min
Enantiomeric purity = 97.5% ee

Conditions of Chiral HPLC:
Mobile phase: EtOH/n-Heptane 75/25 v/v
Column: CHIRALPAK AD-H 5 µm, 0.46*25 cm
Flow: 0.35 mL/min (44 bar)
UV detector: wavelength set at 280 nm

### Example 4: (R)-4-(2-(1-phenyl-4,5,6,7-tetrahydro-1H-indazol-4-yl)ethyl)morpholine N-oxide hydrochloride.

To a (R)-4-(2-(1-phenyl-4,5,6,7-tetrahydro-1H-indazol-4-yl)ethyl)morpholine N-oxide (0.26 g, 0.80 mmol) dissolved in acetone (4 mL) was added HCl 4N in dioxane (0.19 mL) to yield (R)-4-(2-(1-phenyl-4,5,6,7-tetrahydro-1H-indazol-4-yl)ethyl)morpholine N-oxide hydrochloride as a solid (0.15 g, 0.40 mmol, 50%).

Solid beige. m.p. 169-174°C

¹H NMR (300 MHz, DMSO-*d₆*) δ: 12.40 (br s, 1 H), 7.67 (s, 1 H), 7.52 (m, 4H), 7.36 (m, 1 H), 3.96 (m, 4H), 3.89 (m, 2H), 3.74 (m, 4H), 2.74 (m, 3H), 2.26 (m, 1 H), 1.93 (m, 3H), 1.63 (m, 1 H), 1.42 (m, 1 H).

### Example 5: (S)-4-(2-(1-phenyl-4,5,6,7-tetrahydro-1H-indazol-4-yl)ethyl)morpholine N-oxide.

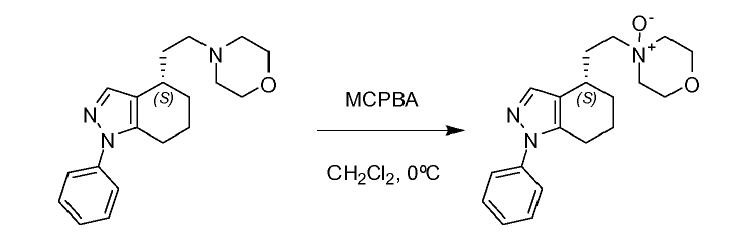

To a solution of (S)-4-(2-(1-phenyl-4,5,6,7-tetrahydro-1H-indazol-4-yl)ethyl) morpholine (277 mg, 0.89 mmol) in CH₂Cl₂ (20 mL) was added portion wise m-chloroperbenzoic acid (∼70% purity, 261 mg, 1.06 mmol) and stirred at room temperature for 5 hours, monitoring the reaction by TLC. The solution was washed twice with NaOH 1 N and water and the organic phase was dried and removed under reduced pressure. The resulting crude was purified by flash chromatography to yield (S)-4-(2-(1-phenyl-4,5,6,7-tetrahydro-1H-indazol-4-yl)ethyl)morpholine N-oxide as an oil (97 mg, 0.30 mmol, 33.0%).
[α]²⁰₄₃₆ = +9.8 (c=1, MeOH)
Retention time = 66.40 min
Enantiomeric purity = 99.5% ee
Conditions of Chiral HPLC:
Mobile phase: EtOH/n-Heptane 75/25 v/v
Column: CHIRALPAK AD-H 5 µm, 0.46*25 cm
Flow: 0.35 mL/min (44 bar)
UV detector: wavelength set at 280 nm

### Example 6: Synthesis of (S)-4-(2-(1-phenyl-4,5,6,7-tetrahydro-1H-indazol-4-yl)ethyl)morpholine N-oxide hydrochloride.

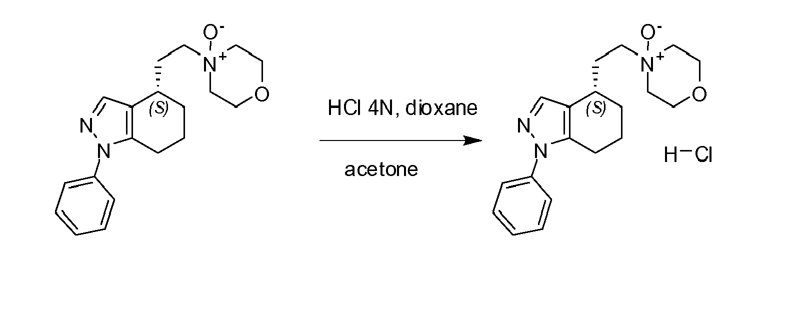

To a (S)-4-(2-(1-phenyl-4,5,6,7-tetrahydro-1H-indazol-4-yl)ethyl)morpholine N-oxide (0.48 g, 1.48 mmol) dissolved in acetone (4 mL) was added HCl 4N in dioxane (0.37 mL) to yield (S)-4-(2-(1-phenyl-4,5,6,7-tetrahydro-1H-indazol-4-yl)ethyl)morpholine N-oxide hydrochloride as a solid (0.26 g, 0.72 mmol, 49%).
Beige solid. m.p. 158-162°C
¹H NMR (300 MHz, DMSO-*d₆*) δ: 12.37 (s, 1 H), 7.68 (s, 1 H), 7.53 (m, 4H), 7.37 (m, 1 H), 3.96 (m, 4H), 3.93 - 3.84 (m, 2H), 3.74 (br s, 4H), 2.75 (m, 3H), 2.28 (m, 1 H), 1.95 (m, 3H), 1.65 (m, 1 H), 1.43 (m, 1 H).

### Example 7: Synthesis of 4-(2-(1-phenyl-4,5,6,7-tetrahydro-1H-indazol-4-yl)ethyl)morpholin-3-one.

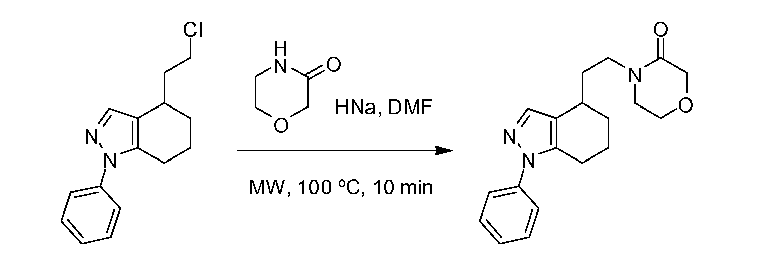

NaH (38 mg of 55-65% dispersion, 0.96 mmol) was added to a solution of morpholin-3-one (42.5 mg, 0.42 mmol) in DMF (2 mL) under argon at 0°C and the mixture was stirred for 30 minutes and 4-(2-chloroethyl)-1-phenyl-4,5,6,7-tetrahydro-1H-indazole (100 mg, 0.38 mmol) was added to the previous mixture and allowed to reach room temperature. The mixture was microwave irradiated at 100 °C (power set point 150 W; hold time 10 min). The solvent was removed at reduced pressure and the resulting crude was diluted in ethyl acetate. The organic phase was washed with acid water and then with basic water. The organic phase was dried and the solvent was removed at reduced pressure. The crude was purified by flash chromatography to yield 4-(2-(1-phenyl-4,5,6,7-tetrahydro-1H-indazol-4-yl)ethyl)morpholin-3-one as an orange oil (37 mg, 0.113 mmol, 29%).
¹H NMR (400 MHz, CDCl₃) δ 7.55 (s, 1 H), 7.52-7.39 (m, 4H), 7.35-7.27 (m, 1H), 4.18 (s, 2H), 3.90 (t, J= 5.1 Hz, 2H), 3.65 (m, 1 H), 3.51 (m, 1 H), 3.41 (q, J= 5.2 Hz, 2H), 2.77 (m, 1 H), 2.71 (m, 2H), 2.10-1.95 (m, 3H), 1.76-1.65 (m, 2H), 1.47 (m, 1 H).

### Example 8: Synthesis of (R)-4-(2-(1-phenyl-4,5,6,7-tetrahydro-1H-indazol-4-yl)ethyl)morpholin-3-one.

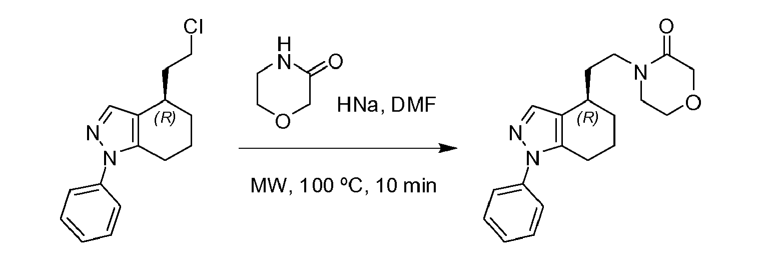

The title compound was prepared from (R)-4-(2-chloroethyl)-1-phenyl-4,5,6,7-tetrahydro-1H-indazole (200 mg, 0.77 mmol), NaH (76 mg of 55-65% dispersion, 1.91 mmol) and morpholin-3-one (85 mg, 0.84 mmol) in DMF (2mL) and the mixture was microwave irradiated at 100 °C (power set point 150 W; hold time 10 min). The crude was purified by flash chromatography to yield (R)-4-(2-(1-phenyl-4,5,6,7-tetrahydro-1H-indazol-4-yl)ethyl)morpholin-3-one (70.1 mg, 0.21 mmol, 28%).
¹H NMR (300 MHz, CDCl₃) δ 7.54 (s, 1 H), 7.47 (m, 4H), 7.32 (m, 1 H), 4.18 (s, 2H), 3.90 (t, J= 5.1 Hz, 2H), 3.67 (m, 1 H), 3.55-3.35 (m, 3H), 2.71 (m, 3H), 2.02 (m, 3H), 1.70 (m, 2H), 1.48 (m, 1 H)
[α]²⁰_{D} = +1.6 (c=0.5, MeOH)
Enantiomeric purity = 92% ee
Column: (Chiralcel OD-H)

### Example 9: Synthesis of (S)-4-(2-(1-phenyl-4,5,6,7-tetrahydro-1H-indazol-4-yl)ethyl)morpholin-3-one.

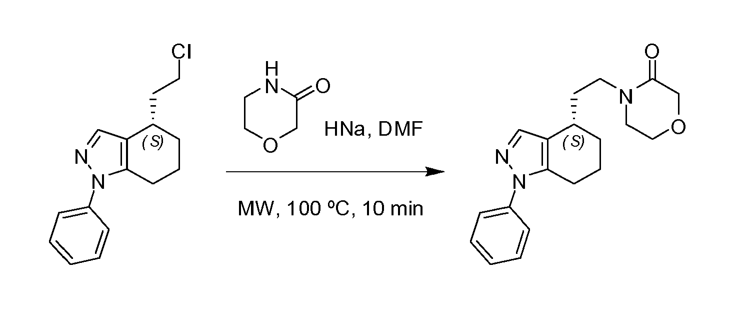

The title compound was prepared from (S)-4-(2-chloroethyl)-1-phenyl-4,5,6,7-tetrahydro-1H-indazole (200 mg, 0.77 mmol), NaH (76 mg of 55-65% dispersion, 1.91 mmol) and morpholin-3-one (85 mg, 0.84 mmol) in DMF (2mL) and the mixture was microwave irradiated at 100 °C (power set point 150 W; hold time 10 min). The crude was purified by flash chromatography to yield (S)-4-(2-(1-phenyl-4,5,6,7-tetrahydro-1H-indazol-4-yl)ethyl)morpholin-3-one (47mg, 0.14 mmol, 19%).
¹H NMR spectroscopic data are identical to those of the example 8
[α]²⁰_{D} = -0.9 (c=0.5, MeOH)
Enantiomeric purity = 99% ee
Column: (Chiralcel OD-H)

### Example 10: Synthesis of 2-(2-(1-phenyl-4,5,6,7-tetrahydro-1H-indazol-4-yl)ethylamino)ethanol.

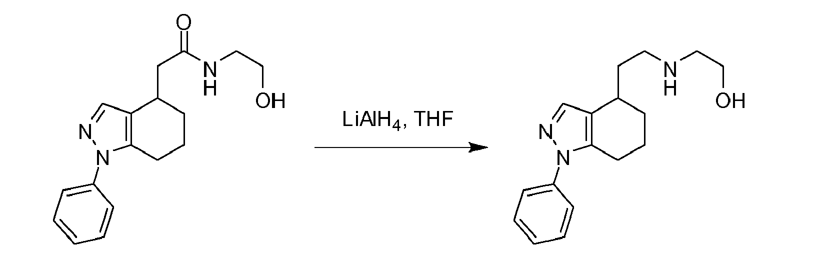

N-(2-hydroxyethyl)-2-(1-phenyl-4,5,6,7-tetrahydro-1H-indazol-4-yl)acetamide (0.85 g, 2.98 mmol) was added to a stirred suspension of LiAlH4 (0.57 g, 14.8 mmol) in dry THF (10 mL) at 0 °C and the mixture was refluxed for 5 h. The reaction mixture was cooled and the excess of hydride was decomposed with water and sodium hydroxide. The mixture was filtered and washed with ethyl acetate. The organic phase was washed with water, dried and removed under reduced pressure to yield 2-(2-(1-phenyl-4,5,6,7-tetrahydro-1H-indazol-4-yl)ethylamino)ethanol (0.78 g, 2.74 mmol, 92%) as a yellowish oil.
¹H NMR (400 MHz, CDCl₃) δ 7.52 (s, 1 H), 7.50-7.41 (m, 4H), 7.31 (m, 1 H), 3.69 (t, J= 5.1 Hz, 2H), 2.90-2.76 (m, 5H), 2.70 (m, 2H), 1.97 (m, 3H), 1.69 (m, 2H), 1.42 (m, 1 H).

### Example 11: Synthesis of 2-(2-(1-phenyl-4,5,6,7-tetrahydro-1H-indazol-4-yl)ethylamino)ethanol hydrochloride.

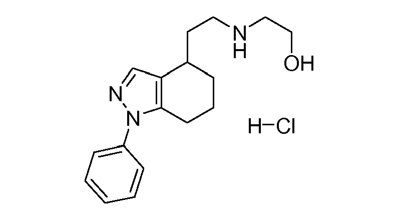

To 2-(2-(1-phenyl-4,5,6,7-tetrahydro-1H-indazol-4-yl)ethylamino)ethanol (0.78 g, 2.74 mmol) dissolved in isopropanol (4 mL) was added HCl 4N in dioxane (0.68 mL) to yield 2-(2-(1-phenyl-4,5,6,7-tetrahydro-1H-indazol-4-yl)ethylamino)ethanol hydrochloride as a beige solid (0.41 g, 1.28 mmol, 47%). m.p. 158-161°C.

### Example 12: Synthesis of (R)-2-(2-(1-phenyl-4,5,6,7-tetrahydro-1H-indazol-4-yl)ethylamino)ethanol.

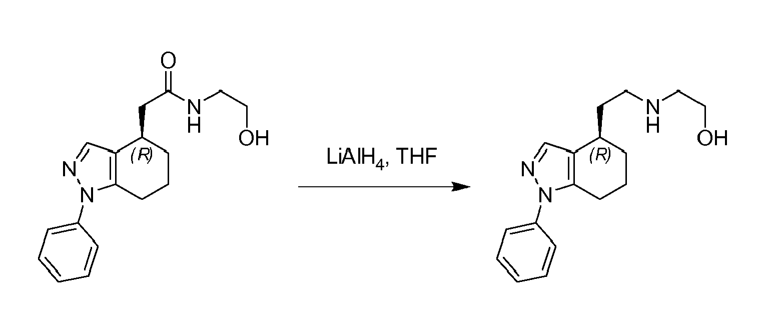

The title compound was prepared according to the general procedure provided in example 9 from (R)-N-(2-hydroxyethyl)-2-(1-phenyl-4,5,6,7-tetrahydro-1H-indazol-4-yl)acetamide (0.52 g, 1.73 mmol), LiAlH4 (0.40 g, 10.93 mmol) in dry THF (80 mL). (R)-2-(2-(1-phenyl-4,5,6,7-tetrahydro-1H-indazol-4-yl)ethylamino)etanol was obtained as an amber oil (0.42 g, 1.49 mmol, 86%).
¹H NMR (300 MHz, CDCl₃) δ 7.52 (s, 1 H), 7.50-7.41 (m, 4H), 7.31 (m, 1 H), 3.67 (t, J= 5.2 Hz, 2H), 2.85-2.84 (m, 5H), 2.71 (m, 2H), 1.96 (m, 3H), 1.67 (m, 2H), 1.44 (m, 1 H).
[α]²⁰_{D} = +7.1 (c=1, MeOH).

### Example 13: Synthesis of (R)-2-(2-(1-phenyl-4,5,6,7-tetrahydro-1H-indazol-4-yl)ethylamino)ethanol hydrochloride.

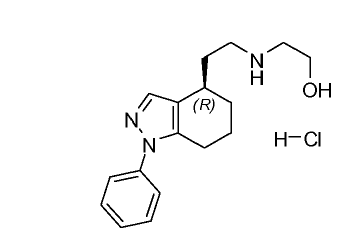

To (R)-2-(2-(1-phenyl-4,5,6,7-tetrahydro-1H-indazol-4-yl)ethylamino)ethanol (0.53 g, 1.88 mmol) dissolved in isopropanol (3 mL) was added HCl 4N in dioxane (0.47 mL) to yield (R)-2-(2-(1-phenyl-4,5,6,7-tetrahydro-1-H-indazol-4-yl)ethylamino)ethanol hydrochloride as a beige solid (0.26 g, 1.21 mmol, 64%). m.p. 174-176°C.
[α]²⁰_{D} +4.8 (c=1, MeOH).
Enantiomeric putity 97%ee
Column: (Chiralpak AD-H)

### Example 14: Synthesis of (S)-2-(2-(1-phenyl-4,5,6,7-tetrahydro-1H-indazol-4-yl)ethylamino)ethanol.

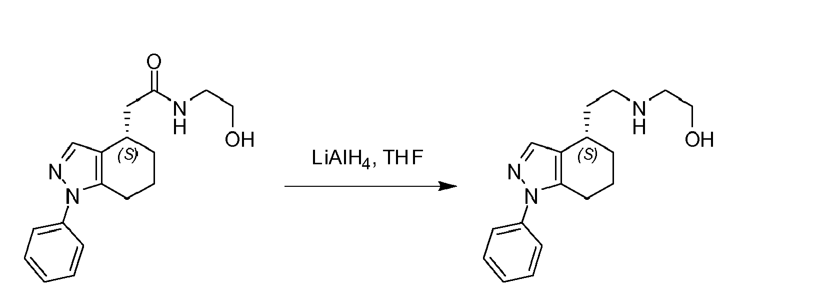

The title compound was prepared according to the general procedure provided in example 9 from (S)-N-(2-hydroxyethyl)-2-(1-phenyl-4,5,6,7-tetrahydro-1H-indazol-4-yl)acetamide (1.01 g, 3.39 mmol), LiAlH4 (0.64 g, 16.6 mmol) in dry THF (100 mL). (S)-2-(2-(1-phenyl-4,5,6,7-tetrahydro-1H-indazol-4-yl)ethylamino)etanol was obtained as an yellowish oil (0.81 g, 2.82 mmol, 83%).
¹H NMR spectroscopic data are identical to those of the example 11
[α]²⁰_{D} = -4.4 (c=0.5, MeOH)
Column: (Chiralpak AD-H).

### Example 15: Synthesis of (S)-2-(2-(1-phenyl-4,5,6,7-tetrahydro-1H-indazol-4-yl)ethylamino)ethanol hydrochloride.

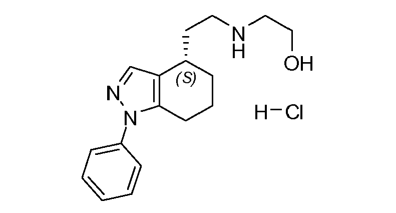

To (S)-2-(2-(1-phenyl-4,5,6,7-tetrahydro-1H-indazol-4-yl)ethylamino)ethanol (56 mg, 0.19 mmol) dissolved in isopropanol (1 mL) was added HCl 4N in dioxane (0.047 mL) to yield (S)-2-(2-(1-phenyl-4,5,6,7-tetrahydro-1H-indazol-4-yl)ethylamino)ethanol hydrochloride as a white solid (31.1 mg, 0.096 mmol, 48%). m.p. 174-175°C.
[α]²⁰_{D} = -5.1 (c=1, MeOH)
Enantiomeric purity = 99%ee
Column: (Chiralpak AD-H)

### Example 16: Synthesis of N-(2-hydroxyethyl)-N-(2-(1-phenyl-4,5,6,7-tetrahydro-1 H-indazol-4-yl)ethyl)formamide.

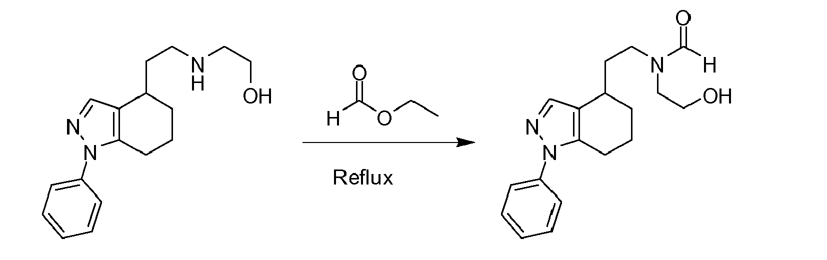

A mixture of 2-(2-(1-phenyl-4,5,6,7-tetrahydro-1H-indazol-4-yl)ethylamino)ethanol (0.14 g, 0.49 mmol) and an excess of ethyl formate was refluxed for 2 hours. The excess of ethyl formate was removed at reduced pressure. The residue was diluted with ethyl acetate and washed with water, dried and removed under reduced pressure and the resulting material was purified by flash chromatography to yield N-(2-hydroxyethyl)-N-(2-(1-phenyl-4,5,6,7-tetrahydro-1H-indazol-4-yl)ethyl)formamide (128 mg, 0.41 mmol, 28%) as a thick paste.

### Example 17: Synthesis of (R)-N-(2-hydroxyethyl)-N-(2-(1-phenyl-4,5,6,7-tetrahydro-1H-indazol-4-yl)ethyl)formamide.

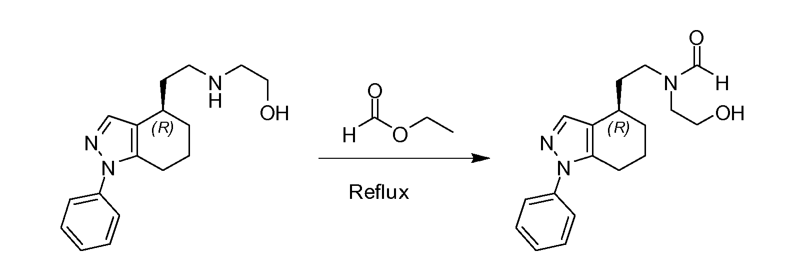

The title compound was prepared according to the general procedure provided in example 15 from (R)-2-(2-(1-phenyl-4,5,6,7-tetrahydro-1H-indazol-4-yl)ethylamino)ethanol (350 mg, 1.22 mmol) and an excess of ethyl formate. (R)-N-(2-hydroxyethyl)-N-(2-(1-phenyl-4,5,6,7-tetrahydro-1H-indazol-4-yl)ethyl)formamide (136 mg, 0.43 mmol, 36%) was obtained as a thick paste.

Enantiomeric purity >98%ee. The enantiomeric excess was determined by NMR using (R)-(-)-2,2,2-trifluoro-1-(9-anthryl)-ethanol as chiral solvating agent in CDCl₃.

### Example 18: Synthesis of (S)-N-(2-hydroxyethyl)-N-(2-(1-phenyl-4,5,6,7-tetrahydro-1H-indazol-4-yl)ethyl)formamide.

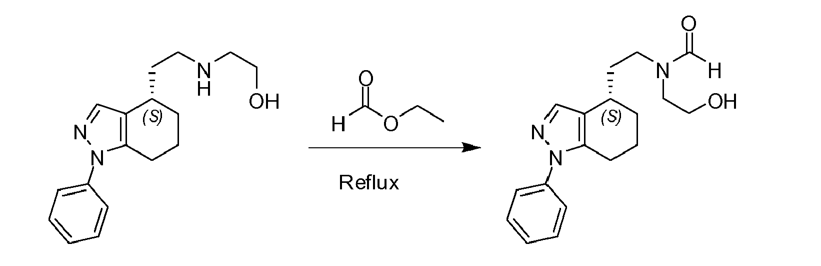

The title compound was prepared according to the general procedure provided in example 15 from (S)-2-(2-(1-phenyl-4,5,6,7-tetrahydro-1H-indazol-4-yl)ethylamino)ethanol (410 mg, 1.44 mmol) and an excess of ethyl formate. (S)-N-(2-hydroxyethyl)-N-(2-(1-phenyl-4,5,6,7-tetrahydro-1H-indazol-4-yl)ethyl)formamide (110 mg, 0.35 mmol, 24%) was obtained as a thick paste.
Enantiomeric purity >98%ee. The enantiomeric excess was determined by NMR using (R)-(-)-2,2,2-trifluoro-1-(9-anthryl)-ethanol as chiral solvating agent in CDCl₃.

### Example 19: Synthesis of 4-(4-(2-morpholinoethyl)-4,5,6,7-tetrahydro-1H-indazol-1-yl)phenol.

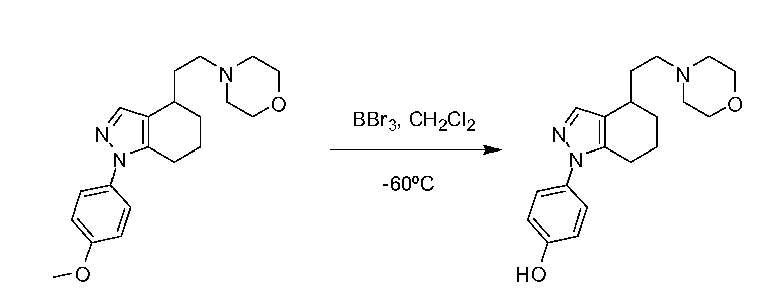

To a solution of 4-(2-(1-(4-methoxyphenyl)-4,5,6,7-tetrahydro-1H-indazol-4-yl)ethyl)morpholine (60 mg, 0.176 mmol) in dichloromethane (3 mL) cooled at -60°C was added BBr₃ (0.53 mmol, 0.53 mL of 1 M BBr₃ in dichloromethane) and the mixture was stirred at room temperature for 24 hours. The reaction mixture was cooled, diluted with water and dichloromethane. The organic phase was separated, washed with sodium bicarbonate (saturated solution), water and evaporated. The resulting material was purified by flash chromatography to yield 4-(4-(2-morpholinoethyl)-4,5,6,7-tetrahydro-1H-indazol-1-yl)phenol (10 mg, 0.03 mmol, 17%) as a thick paste.
¹H NMR (300 MHz, CDCl₃) δ 7.48 (s, 1 H), 7.23 (d, *J* = 8.8 Hz, 2H), 6.78 (d, *J* = 8.8 Hz, 2H), 3.75 (t, *J* = 4.6 Hz, 4H), 2.76 (s, 1 H), 2.65-2.41 (m,8H), 1.92 (m, 3H), 1.68 (m, 2H), 1.42 (m, 1 H).

### BIOLOGICAL ACTIVITY EXAMPLES

Some representative compounds of the invention were tested for their activity as sigma (sigma-1) inhibitors. The following protocols were followed:

### Sigma-1

Brain membrane preparation and binding assays for the σ1-receptor were performed as described (DeHaven-Hudkins et al., 1992) with some modifications. In brief, guinea pig brains were homogenized in 10 vols. (w/v) of Tris-HCl 50 mM 0.32 M sucrose, pH 7.4, with a Kinematica Polytron PT 3000 at 15000 r.p.m. for 30 s. The homogenate was centrifuged at 1000g for 10 min at 4°C and the supernatants collected and centrifuged again at 48000g for 15 min at 4°C. The pellet was resuspended in 10 volumes of Tris-HCl buffer (50 mM, pH 7.4), incubated at 37°C for 30 min, and centrifuged at 48000g for 20 min at 4°C. Following this, the pellet was resuspended in fresh Tris-HCl buffer (50 mM, pH 7.4) and stored on ice until use.

Each assay tube contained 10 µL of [³H](+)-pentazocine (final concentration of 0.5 nM), 900 µL of the tissue suspension to a final assay volume of 1 mL and a final tissue concentration of approximately 30 mg tissue net weight/mL. Non-specific binding was defined by addition of a final concentration of 1 µM haloperidol. All tubes were incubated at 37°C for 150 min before termination of the reaction by rapid filtration over Schleicher & Schuell GF 3362 glass fibre filters [previously soaked in a solution of 0,5% polyethylenimine for at least 1 h]. Filters were then washed with four times with 4 mL of cold Tris-HCl buffer (50 mM, pH 7.4). Following addition of scintillation cocktail, the samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).

### References

DeHaven-Hudkins, D. L., L.C. Fleissner, and F. Y. Ford-Rice, 1992, Characterization of the binding of [3H](+)pentazocine to σ recognition sites in guinea pig brain, Eur. J. Pharmacol. 227, 371-378*.*

Lowry, O.H., N.J. Rosebrough, A.L. Farr, and R.J. Randall, 1951, Protein measurement with the Folin phenol reagent, J. Biol. Chem, 193, 265.

The results are summarized in the following table I:

**Table I**

| compound no. | binding sigma-1 % Inh (1µM) |
|---|---|
| 4-(2-(1-phenyl-4,5,6,7-tetrahydro-1H-indazol-4-yl) ethyl)morpholine N-oxide (Example 1) | 60 |
| (R)-4-(2-(1-phenyl-4,5,6,7-tetrahydro-1 H-indazol-4-yl) ethyl)morpholine N-oxide (Example 3) | 76.4 |
| (S)-4-(2-(1-phenyl-4,5,6,7-tetrahydro-1 H-indazol-4-yl)ethyl)morpholine N-oxide (Example 5) | 98.0 |

## Claims

1. A compound of the formula (I) 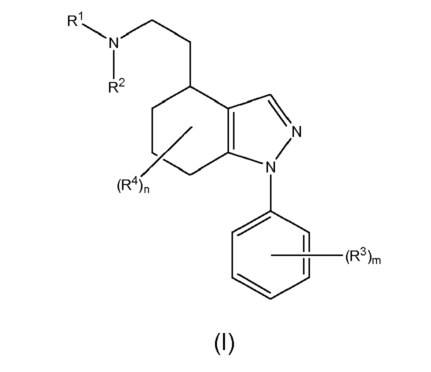 wherein
R¹ is selected from hydrogen or-CHO and R² is hydroxyethyl; or
R¹ and R² together with the nitrogen atom to which they are attached form a morpholinyl ring or a 2,3-dihydro-[1,4]oxazin-4-yl ring, said rings being optionally substituted with one or two groups selected from C₁-₆alkyl, hydroxy, C₁₋₆alkoxy or oxo groups;
R³ is independently halogen, hydroxy or C₁₋₆alkoxy;
R⁴ is independently hydroxy or C₁₋₆alkoxy;
n and m are independently selected from 0, 1, and 2;
or a pharmaceutically acceptable N-oxide, salt, isomer, prodrug or solvate thereof;
with the proviso that the compound 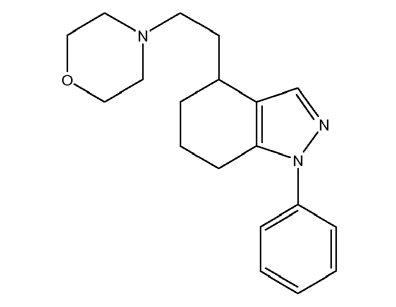 and its salts are excluded.

2. Compound according to claim 1, wherein said compound has the formula (Ia): 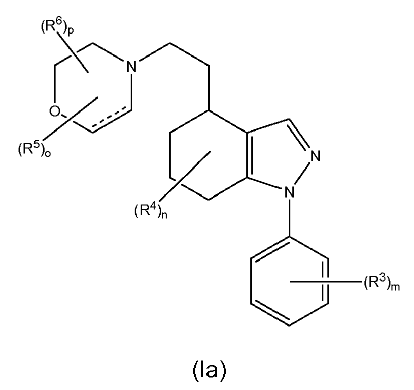 wherein
the dashed line (represented by - - - - -) represents an optional double bond;
R³ is independently halogen, hydroxy or C₁₋₆alkoxy;
R⁴ is independently hydroxy or C₁₋₆alkoxy;
R⁵ is independently C₁₋₆alkyl, hydroxy or C₁₋₆alkoxy;
R⁶ is oxo;
n, m and o are independently selected from 0, 1, and 2;
p is selected from 0, 1 and 2, with the proviso that o + p is ≤ 2;
or a pharmaceutically acceptable N-oxide, salt, isomer, prodrug or solvate thereof;
with the proviso that the compound 4,5,6,7-tetrahydro-4-(2-(morpholin-4-yl)ethyl)-1-phenyl-1H-indazole and its salts are excluded.

3. Compound according to any of claims 1 to 2, wherein said compound is selected from a compound of formula (Iaa), (lab) or (lac): 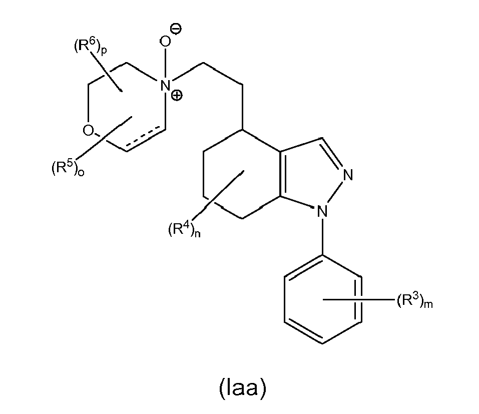 wherein
the dashed line (represented by - - - - -) represents an optional double bond;
R³ is independently halogen, hydroxy or C₁₋₆alkoxy;
R⁴ is independently hydroxy or C₁₋₆alkoxy;
R⁵ is independently C₁₋₆alkyl, hydroxy or C₁₋₆alkoxy;
R⁶ is oxo;
n, m and o are independently selected from 0, 1, and 2;
p is selected from 0, 1 and 2, with the proviso that o + p is ≤ 2;
or a pharmaceutically acceptable salt, isomer, prodrug or solvate thereof; 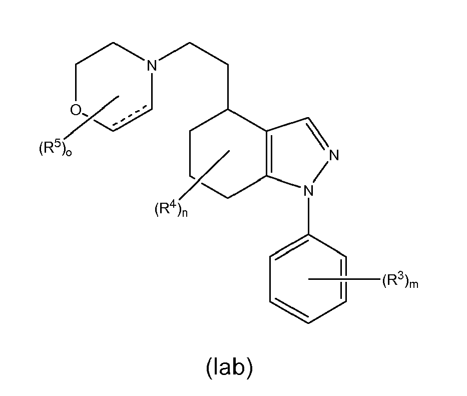 wherein
the dashed line (represented by - - - - -) represents an optional double bond;
R³ is independently halogen, hydroxy or C₁₋₆alkoxy;
R⁴ is independently hydroxy or C₁₋₆alkoxy;
R⁵ is independently C₁₋₆alkyl, hydroxy or C₁₋₆alkoxy;
n, m and o are independently selected from 0, 1, and 2, with the proviso that n + m + o is ≥ 1;
or a pharmaceutically acceptable salt, isomer, prodrug or solvate thereof; 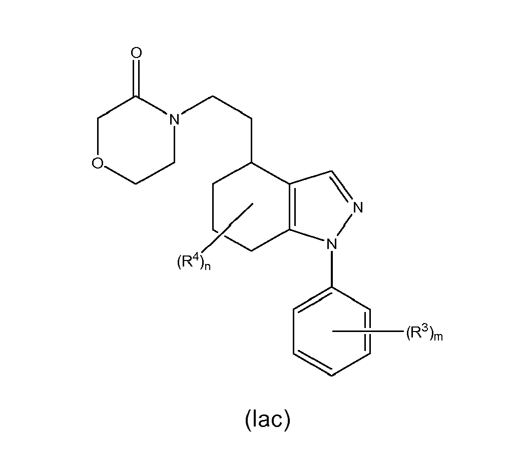 wherein
R³ is independently halogen, hydroxy or C₁₋₆alkoxy;
R⁴ is independently hydroxy or C₁₋₆alkoxy;
n and m are independently selected from 0, 1, and 2;
or a pharmaceutically acceptable salt, isomer, prodrug or solvate thereof.

4. Compound according to claim 1, wherein said compound has the formula (Ib): 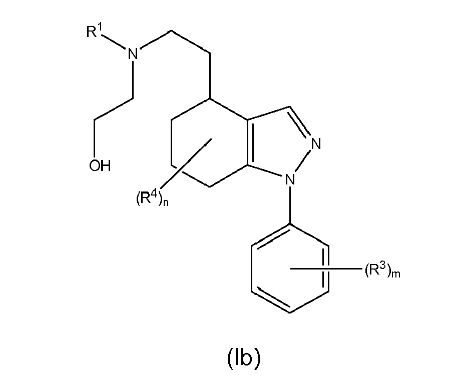 wherein
R¹ is selected from hydrogen or-CHO;
R³ is independently halogen, hydroxy or C₁₋₆alkoxy;
R⁴ is independently hydroxy or C₁₋₆alkoxy;
n and m are independently selected from 0, 1, and 2;
or a pharmaceutically acceptable N-oxide, salt, isomer, prodrug or solvate thereof.

5. Compound of formula (I) according to claim 1, wherein said compound is selected from:
- 4-(2-(1-phenyl-4,5,6,7-tetrahydro-1H-indazol-4-yl)ethyl)morpholine N-oxide;
- 4-(2-(1-(3,4-dichlorophenyl)-4,5,6,7-tetrahydro-1H-indazol-4-yl)ethyl)morpholine N-oxide;
- 2,6-dimethyl-4-(2-(1-phenyl-4,5,6,7-tetrahydro-1H-indazol-4-yl)ethyl)morpholine N-oxide;
- (2R,6S)-2,6-dimethyl-4-(2-(1-phenyl-4,5,6,7-tetrahydro-1H-indazol-4-yl)ethyl)morpholine N-oxide;
- 4-(2-(1-(4-fluorophenyl)-4,5,6,7-tetrahydro-1H-indazol-4-yl)ethyl)morpholine N-oxide;
- (R)-4-(2-(1-(4-fluorophenyl)-4,5,6,7-tetrahydro-1H-indazol-4-yl)ethyl)morpholine N-oxide;
- (S)-4-(2-(1-(4-fluorophenyl)-4,5,6,7-tetrahydro-1H-indazol-4-yl)ethyl)morpholine N-oxide;
- 4-(2-(1-(4-fluorophenyl)-4,5,6,7-tetrahydro-1H-indazol-4-yl)ethyl)-2,6-dimethylmorpholine N-oxide;
- (2R,6S)-4-(2-(1-(4-fluorophenyl)-4,5,6,7-tetrahydro-1H-indazol-4-yl)ethyl)-2,6-dimethylmorpholine N-oxide;
- 4-(2-Morpholin-4-yl-ethyl)-1-phenyl-4,5,6,7-tetrahydro-1H-indazol-5-ol;
- 4-(2-Morpholin-4-yl-ethyl)-1-phenyl-4,5,6,7-tetrahydro-1H-indazol-6-ol;
- 4-(2-Morpholin-4-yl-ethyl)-1-phenyl-4,5,6,7-tetrahydro-1H-indazol-7-ol;
- 1-(2-Hydroxy-phenyl)-4-(2-morpholin-4-yl-ethyl)-4,5,6,7-tetrahydro-1H-indazol-5-ol;
- 1-(3-Hydroxy-phenyl)-4-(2-morpholin-4-yl-ethyl)-4,5,6,7-tetrahydro-1H-indazol-5-ol;
- 1-(4-Hydroxy-phenyl)-4-(2-morpholin-4-yl-ethyl)-4,5,6,7-tetrahydro-1H-indazol-5-ol;
- 1-(2-Hydroxy-phenyl)-4-(2-morpholin-4-yl-ethyl)-4,5,6,7-tetrahydro-1H-indazol-6-ol;
- 1-(3-Hydroxy-phenyl)-4-(2-morpholin-4-yl-ethyl)-4,5,6,7-tetrahydro-1H-indazol-6-ol;
- 1-(4-Hydroxy-phenyl)-4-(2-morpholin-4-yl-ethyl)-4,5,6,7-tetrahydro-1H-indazol-6-ol;
- 1-(2-Hydroxy-phenyl)-4-(2-morpholin-4-yl-ethyl)-4,5,6,7-tetrahydro-1H-indazol-7-ol;
- 1-(3-Hydroxy-phenyl)-4-(2-morpholin-4-yl-ethyl)-4,5,6,7-tetrahydro-1H-indazol-7-ol;
- 1-(4-Hydroxy-phenyl)-4-(2-morpholin-4-yl-ethyl)-4,5,6,7-tetrahydro-1H-indazol-7-ol;
- 2-[4-(2-Morpholin-4-yl-ethyl)-4,5,6,7-tetrahydro-indazol-1-yl]-phenol;
- 3-[4-(2-Morpholin-4-yl-ethyl)-4,5,6,7-tetrahydro-indazol-1-yl]-phenol;
- 4-[4-(2-Morpholin-4-yl-ethyl)-4,5,6,7-tetrahydro-indazol-1-yl]-phenol;
- 4-[2-(1-Phenyl-4,5,6,7-tetrahydro-1H-indazol-4-yl)-ethyl]-3,4-dihydro-2H-[1,4]oxazin-2-ol;
- 4-[2-(1-Phenyl-4,5,6,7-tetrahydro-1H-indazol-4-yl)-ethyl]-3,4-dihydro-2H-[1,4]oxazin-3-ol;
- 4-[2-(1-Phenyl-4,5,6,7-tetrahydro-1H-indazol-4-yl)-ethyl]-5,6-dihydro-4H-[1,4]oxazin-2-ol;
- 4-[2-(1-Phenyl-4,5,6,7-tetrahydro-1H-indazol-4-yl)-ethyl]-5,6-dihydro-4H-[1,4]oxazin-3-ol;
- 4-[2-(1-Phenyl-4,5,6,7-tetrahydro-1H-indazol-4-yl)-ethyl]-morpholin-3-one;
- 2-[2-(1-Phenyl-4,5,6,7-tetrahydro-1H-indazol-4-yl)-ethylamino]-ethanol; and
- N-(2-Hydroxy-ethyl)-N-[2-(1-phenyl-4,5,6,7-tetrahydro-1H-indazol-4-yl)-ethyl]-formamide;
or a pharmaceutically acceptable N-oxide, salt, isomer, prodrug or solvate thereof.

6. Compound of formula (I) according to claim 5, wherein said compound is (S)-4-(2-(1-phenyl-4,5,6,7-tetrahydro-1H-indazol-4-yl)ethyl)morpholine N-oxide hydrochloride.

7. A process for the preparation of a compound of formula (I) as defined in any of claims 1 to 6 or a pharmaceutically acceptable N-oxide, salt, isomer, prodrug or solvate thereof, which comprises the coupling of a compound of formula (II): 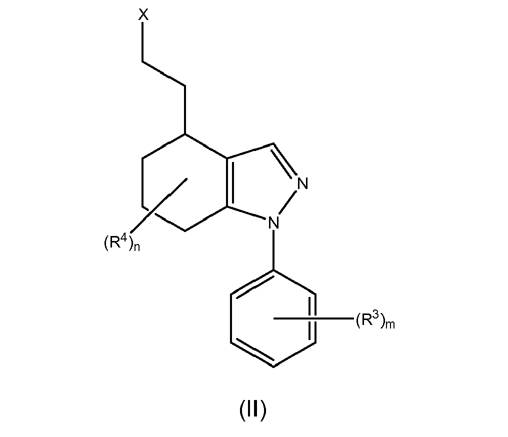 in which R³-R⁴, m and n are as defined above in formula (I), and X is a leaving group, preferably chlorine or pyridinium, with a compound of formula (III):
HNR¹R² (III)
in which R¹ and R² are as defined above in formula (I).

8. A pharmaceutical composition comprising at least one compound of formula (I) as defined in any of claims 1 to 6, or a pharmaceutically acceptable N-oxide, salt, isomer, prodrug or solvate thereof and a pharmaceutically acceptable carrier, adjuvant or vehicle.

9. A compound of formula (I) as defined in any of claims 1 to 6 for use as a medicament.

10. A compound of formula (I) as defined in any of claims 1 to 6 for use for the treatment and/or prophylaxis of a sigma receptor-mediated disease or condition.

11. A compound of formula (I) as defined in any of claims 1 to 6 for use for the treatment and/or prophylaxis of a sigma receptor-mediated disease or condition selected from the group consisting of diarrhoea; migraine; obesity; elevated trigyceride levels; chylomicronemia; dysbetalipoproteinemia; hyperlipoproteinemia, hyperlipidemia; mixed hyperlipidemia; hypercholesterolemia; lipoprotein disorders; hypertriglyceridemia; sporadic hypertriglyceridemia; inherited hypertriglyceridemia; and dysbetalipoproteinemia; arthritis; hypertension; arrhythmia; ulcer; learning, memory and attention deficits; cognition disorders; neurodegenerative diseases; demyelinating diseases; addiction to drugs and chemical substances; tardive diskinesia; ischemic stroke; epilepsy; stroke; stress; cancer; psychotic conditions; inflammation; and autoimmune diseases.

12. A compound of formula (I) as defined in claim 11 wherein the phycotic condition is depression, anxiety or schizophrenia.

13. A compound of formula (I) as defined in claim 11 wherein the addiction to drugs and chemical substances includes addiction to cocaine, amphetamine, ethanol or nicotine.

14. A compound of formula (I) as defined in any of claims 1 to 6 for use for the treatment and/or prophylaxis of pain, especially neuropathic pain, inflammatory pain or other pain conditions involving allodynia and/or hyperalgesia.

15. A compound of formula (I) as defined in any of claims 1 to 6 for use as a pharmacological tool.
